# EUROPÄISCHE PATENTSCHRIFT

(11) **EP 2 574 377 B1**
(45) Veröffentlichungstag und Bekanntmachung des Hinweises auf die Patenterteilung: **13.09.2017**
(21) Anmeldenummer: 12174380.1
(22) Anmeldetag: 29.06.2012
(51) Int. Cl.: A61B 5/024, A61B 5/00, A63B 71/06, A63B 24/00, A63B 22/00

(54) **Stationäres Übungsgerät zur körperlichen Ertüchtigung, insbesondere Trainingsrad**
Stationary exercise device for physical training, in particular exercise bicycle
Appareil stationnaire d'exercice pour l'entraînement physique, notamment vélo d'appartement

(30) Priorität: 29.09.2011 DE 102011114521
(43) Veröffentlichungstag der Anmeldung: 03.04.2013
(73) Patentinhaber: PROTOKON Gyártó, Fejlesztö és Kereskedö Kft., 6200 Kiskörös (HU)
(72) Erfinder: Fischer, Andreas, 90482 Nürnberg (DE)
(74) Vertreter: Blaumeier, Jörg

(56) Entgegenhaltungen:
- EP-A1- 1 878 473
- EP-A2- 0 174 747
- WO-A2-2007/000774
- DE-U1- 20 113 786
- US-A- 4 919 416

## Beschreibung

Die Erfindung betrifft ein stationäres Übungsgerät zur körperlichen Ertüchtigung, insbesondere ein Trainingsrad, umfassend ein Gestell mit einer vom Übenden zu bewegenden oder selbst angetriebenen und mit dem Übenden zusammenwirkenden Bewegungseinheit, einen oder mehrere der Bewegungseinheit und/oder dem Übenden zugeordnete Sensoren zur Erfassung von Messwerten, sowie eine Recheneinheit zur Ermittlung eines oder mehrerer messwertbezogener Informationen, die an einer gestellseitigen Anzeigeeinrichtung ausgegeben werden.

Der Fitnessgedanke durchlebt gerade in den letzten Jahren einen starken Wandel von einem eher kraftorientierten Training hin zu einem gesundheitlich orientierten Training. Verwendet werden hierzu häufig stationäre Übungsgeräte, die insbesondere dem Ausdauertraining dienen. Das prominenteste Beispiel eines solchen stationären Übungsgeräts ist ein Trainingsrad, mit einem Gestell, einem Sattel, auf dem der Übende sitzt und einem entsprechenden Lenker sowie Pedalen, über die eine Schwungmasse oder Ähnliches bewegt wird. Aber auch Laufbänder, die angetrieben werden und deren Geschwindigkeit und Neigung variiert werden kann, auf welchen der Übende läuft, sind hierunter zu nennen.

Im Rahmen des gesundheitlich orientierten Trainings werden verschiedene Messwerte unter Verwendung eines oder mehrerer geeigneter Sensoren aufgenommen, welche Messwerte einerseits am Übungsgerät selbst abgegriffen werden, andererseits aber auch am Übenden selbst. Während am Gerät beispielsweise die Trittfrequenz oder die Drehzahl über einen geeigneten Drehzahlmesser oder einen Reed-Kontakt, die Leistung in Watt über geeignete Dehnungsmessstreifen, eine Sensorik oder andere Messverfahren wie auch ein gewisses Widerstandslevel über die Einstellung eines entsprechenden Übersetzungsverhältnisses, einer Steigung und Ähnliches über Messsensorik erfasst wird, wird als personenbezogener Messwert zumeist die Herzfrequenz, z. B. über eine vom Übenden getragene Pulsuhr oder einen Brustgurt erfasst.

Insbesondere aus den personenbezogenen Messwerten, also vornehmlich der Herzfrequenz, in Verbindung mit weiteren vom Benutzer einzugebenden, also in einer Recheneinrichtung zu hinterlegenden Daten, kann eine bestimmte Herzfrequenzzone ermittelt werden, in welcher die Herzfrequenz liegen sollte, um ein bestimmtes Trainingsziel zu erreichen. Anhand der einzugebenden Nutzerdaten (z. B. Geschlecht, Alter, Gewicht, Trainingsaktivität etc.) wird eine maximale Herzfrequenz berechnet, anhand welcher sich sodann bestimmte, Frequenzbereiche erfassende Trainingszonen ermitteln lassen, welche zur Trainingssteuerung herangezogen werden. Üblicherweise werden fünf Haupttrainingszonen definiert, die bestimmte Trainingsziele definieren. Diese sind in Prozentabschnitte bezogen auf die maximale persönliche Herzfrequenz definiert und reichen von einer Gesundheitszone im Bereich von 50 - 60 % der maximalen Herzfrequenz über eine Fettverbrennungszone (60 - 70 %), einer aerobe Zone (70 - 80 %) und einer anaeroben Zone (80 - 90 %) bis zu einer "Roten Zone", also einer Warnzone, die von 90 - 100 % der maximalen Herzfrequenz reicht. Je nach gewünschtem Trainingsziel kann nun der Übende seine Tätigkeit so einstellen, dass er mit seiner gemessenen Ist-Herzfrequenz in der gewünschten Trainingszone liegt.

Die Erfassung und Anzeige der Herzfrequenz erfolgt bei so genannten Einzelplatzgeräten über ein individuelles System, beispielsweise unter Verwendung einer Pulsuhr, die der Übende trägt. Diese dient einerseits zur Erfassung der sonstigen einzugebenden Daten, andererseits auch zur Erfassung der Ist-Herzfrequenz und dient auch als Anzeigeeinrichtung, an der der Übende die Informationen ablesen kann. Die sonstigen Trainingsdaten (Drehzahl, Leistung etc.) werden geräteseitig erfasst und an einer separaten Anzeigeeinrichtung wiedergegeben.

Für einen Trainer oder Instruktor, der insbesondere im Bereich der Gruppenfitness, die zunehmend attraktiv wird, das Training leitet und, da unterschiedliche Personen zusammen trainieren, individuelle Trainingsanleitungen geben muss, ist eine individuelle Betreuung quasi nicht möglich, da er keinen Zugriff respektive Überblick über die persönlichen Messwerte und damit Daten hat.

Deshalb ist es im Bereich so genannter Gruppen- oder Mehrplatzlösungen bekannt, dass die über das personenbezogene Messgerät, beispielsweise den Brustgurt, erfassten Informationen, also die gemessene Herzfrequenz, an eine zentrale Empfangsstation, die mit einer Recheneinrichtung und einer Anzeigeeinrichtung verbunden ist, gesendet werden. In dieser sind auch die persönlichen Daten der einzelnen Gruppenmitglieder erfasst, denen die gemessenen Herzfrequenzen entsprechend zugeordnet werden, woraus dann die entsprechenden Trainingszonen etc. ermittelt und angezeigt werden. Die Anzeige erfolgt hier also nicht mehr individuell, sondern zentral an einem Bildschirm, an dem der Übungsleiter die Messwerte aller seiner Teilnehmer erfasst. In diesem Fall sieht der Trainer zwar die einzelnen Belastungen und Trainingszonen der Gruppenmitglieder, es findet jedoch keine individuelle Anzeige der Trainingsdaten am Platz des Trainierenden selbst statt. Auch verliert der Trainer jede Kontrolle und Übersicht, wenn er seinen Platz verlässt, um dem einen oder anderen Übenden Hilfestellung zu geben. Auf der anderen Seite ist der einzelne Trainierende nicht in der Lage, sein eigenes Belastungsprofil zu erkennen, da ihm die trainingszonenbezogenen Informationen nicht angezeigt werden, sondern lediglich die gerätebezogenen Informationen wie Trittfrequenz etc.

In EP 1 878 473 A1 ist ein Trainingsgerät beschrieben, bei dem die verbleibende Trainingsdauer, während der das Trainingsgerät vom momentanen Nutzer noch benutzt wird, ermittelt und mittels einer zusätzlichen Anzeigeeinrichtung einer wartenden Person signalisiert wird.

Der Erfindung liegt dabei das Problem zugrunde, ein stationäres Übungsgerät anzugeben, das durch eine verbesserte Informationsdarstellung eine Verbesserung der Trainingssteuerung ermöglicht.

Zur Lösung dieses Problems ein stationäres Übungsgerät nach Patentanspruch 1 vorgesehen.

Erfindungsgemäß sind an dem Übungsgerät zwei separate Anzeigeeinrichtungen vorgesehen, die zu zwei unterschiedlichen Seiten hin ausgerichtet sind, mithin also die Erfassung der dargestellten Informationen durch unterschiedliche Personen ermöglichen. Die erste Anzeigeeinrichtung ist zum Übenden hin gerichtet, an ihr können alle relevanten Daten dargestellt werden, soweit sie über die gerätebezogene Recheneinrichtung ermittelt werden. Dies sind zum einen sämtliche geräteseitig erfassten Trainingsdaten (Trittfrequenz/Drehzahl, Leistung, Widerstand etc.), aber auch die personenbezogenen Daten wie insbesondere die Herzfrequenz und maximale Herzfrequenz respektive eine etwaige abgeleitete Trainingszone etc., je nachdem, welche personenbezogene Information ermittelt wird. Das heißt, dass insoweit eine Kommunikation auch der Sensoren, die die personenbezogenen Daten erfassen, mit der geräteseitigen Recheneinrichtung gegeben ist. Dies natürlich nur, soweit diese Daten erfasst werden, was natürlich je nach Übungsgerät gegebenenfalls unterschiedlich ist. Denn eine parallele Erfassung der gerätebezogenen wie der personenbezogenen Messwerte ist nicht zwingend erforderlich, je nach Gerätetyp kann auch nur der eine oder andere Messwertsatz erfasst und verarbeitet respektive angezeigt werden.

Erfindungsgemäß vorgesehen ist ferner eine zweite Anzeigeeinrichtung, die zur gegenüberliegenden Seite gerichtet ist, mithin also in Richtung des Trainers. An dieser Anzeigeeinrichtung wird wenigstens eine Information, die für den Trainer relevant und entscheidend für etwaige personenbezogenen Trainingskommandos ist, angezeigt. Vornehmlich natürlich handelt es sich bei dieser dort angezeigten Information um eine personenbezogene Information, also z. B. die Herzfrequenz respektive eine davon abgeleitete Information, insbesondere die Trainingszone, in welcher sich der Übende gerade befindet. Eine Ermittlung z. B. einer Trainingszone ist alternativ auch über die geräteseitig erfasste momentane Wattleistung und den FTP-Index (die maximale Leistung, die ein Fahrer im aeroben/anaeroben Stoffwechsel eine Stunde lang leisten kann, der manuell einzugeben ist) möglich, das heißt, dass die Ermittlung und Darstellung von Trainingszonen nicht nur über die Herzfrequenz umsetzbar ist. Die Information über die Trainingszone ist in der Regel eine entscheidende Information für den Trainer, die er zur Optimierung des Trainings benötigt. In jedem Fall wird dem Trainer diese Information ebenfalls unmittelbar am Übungsgerät visualisiert, sodass er diese zentrale Information auch dann erhält, wenn er sich im Raum bewegt. Üblicherweise sind im Rahmen der Gruppenfitness die Übungsgeräte beispielsweise im Halbkreis aufgestellt oder in versetzten Reihen, sodass der Trainer, wenn er vor der Gruppe steht, alle Übungsgeräte von vorne sieht. Damit sieht er auch sämtliche Anzeigeeinrichtungen, die an der Gerätevorderseite, ihm zugewandt, angeordnet sind, sodass er sich sofort einen Überblick über die entsprechenden Informationen verschaffen kann, wenn er an das jeweilige Gerät herantritt.

Das heißt, dass bei dem erfindungsgemäßen Übungsgerät insbesondere die relevanten Informationen, die sowohl für den Trainierenden als auch den Trainer relevant sind (und hierunter insbesondere eben die herzfrequenzbezogenen Informationen) sowohl dem Trainierenden als auch dem Trainer dargestellt werden.

Dabei kann die dem Trainierenden bereitgestellte, angezeigte Informationsbasis wesentlich breiter sein, da ihm, je nach Erfassungstiefe, sämtliche gerätebezogenen Informationen (Trittfrequenz, Widerstand etc.) wie auch seine personenbezogenen Informationen dargestellt werden können. Für den Trainer hingegen ist es ausreichend, einen Leistungsindikator zu erhalten, der eben die persönliche Belastung des Teilnehmers widerspiegelt, also insbesondere die herzfrequenzbezogene Information, sofern diese erfasst wird. Gleichwohl kann dem Trainer natürlich (alternativ oder zusätzlich) auch eine Information hinsichtlich der Trittfrequenz oder der Leistung etc. angezeigt werden, aus welcher der Trainer, gegebenenfalls ohne Kenntnis der Herzfrequenz respektive Trainingszone, aufgrund seiner Erfahrung Informationen bezüglich des Leistungsstandes ableiten kann, um helfend eingreifen zu können.

Entscheidend ist hierbei jedoch in jedem Fall die beiderseitige Informationsdarstellung an dem einen Übungsgerät, die die Erfassung der Informationen sowohl durch den Trainierenden als auch den Trainer ermöglicht.

Erfindungsgemäss ist vorgesehen, dass die an der zweiten Anzeigeeinrichtung ausgebbare Information simultan mit einer gleichen, an der ersten Anzeigeeinrichtung ausgebbaren Information anzeigbar ist. Beispielsweise kann an der zweiten, zum Trainer gerichteten Anzeigeeinrichtung nur eine Information angezeigt werden. Diese ist vom Informationsinhalt her gleich einer an der ersten, zum Trainierenden gerichteten Anzeigeeinrichtung angezeigten Information, das heißt, dass der Übende und der Trainer die gleiche Information erhalten, mithin also bezüglich einer entscheidenden Information den gleichen Informationsstand haben, wobei die Darstellungsform gleich oder unterschiedlich sein kann. Wichtig ist in jedem Fall die simultane Darstellung, sodass zu jedem Zeitpunkt beide den gleichen Informationsstand besitzen.

Selbstverständlich ist es auch möglich, an der ersten und der zweite Anzeigeeinrichtung simultan mehrere gleiche Informationen darzustellen, also beispielsweise eine erste herzfrequenzbezogene Information (z. B. Trainingszone), wie eine zweite gerätebezogene (z. B. Trittfrequenz). Dies hängt letztlich davon ab, welche Informationen konkret ermittelt werden, und welcher Art die verwendeten Anzeigeeinrichtungen sind.

Als erste Anzeigeeinrichtung, die zum Übenden gerichtet ist, wird bevorzugt ein Display, insbesondere Farbdisplay verwendet. Derartige Displays können in unterschiedlichster Größe verbaut werden, sie bieten eine hinreichend große Informationsfläche, um eine Vielzahl von Informationen darzustellen. Darüber hinaus lassen sie bei Auslegung als Touchscreen auch eine unmittelbare Dateneingabe zu. Alternativ kann die Information über eine oder mehrere Farb-LEDs oder andere auffällige Farbindikatoren an den Übenden gegeben werden.

Auch die zweite Anzeigeeinrichtung, also die zum Trainer gewandte Anzeigeeinrichtung, kann ein Display, insbesondere ein Farbdisplay sein. Auch dieses kann in seiner Größe variabel sein, sodass auch an diesem ein entweder eine hinreichende Informationsbreite darstellbar ist, oder, wenn nur eine Information dargestellt wird, diese entsprechend großflächig angezeigt werden kann, sodass sie der Trainer auch aus einer gewissen Distanz sofort erfassen kann. Alternativ zur Verwendung eines Displays, insbesondere eines Farbdisplays, können auch mehrere in unterschiedlichen Farben leuchtende Lichtquellen, insbesondere LEDs verbaut werden. Diese Lichtquellen können aufgrund der unterschiedlichen Farbe entsprechende farbkodierte Informationen wiedergeben, wobei die unterschiedlichen Farben beispielsweise unterschiedlichen Trainingszonen zugeordnet sein können.

Werden als erste und zweite Anzeigeeinrichtungen jeweils Farbdisplays verwendet, so wird bevorzugt die gleiche Information, die an beiden angezeigt wird, jeweils in einer gleichen Farbdarstellung wiedergegeben. Handelt es sich bei dieser Information insbesondere um die Trainingszone, so kann diese auf einfache Weise farbkodiert dargestellt werden. In gleicher Weise ist eine farbkodierte Darstellung aber auch möglich, wenn die gleiche Information an einem Farbdisplay als erster Anzeigeeinrichtung und einer entsprechenden farbigen Lichtquelle als zweiter Anzeigeeinrichtung dargestellt wird.

Als eine solche Farbdarstellung an einem Farbdisplay wird insbesondere der Displayhintergrund farbig dargestellt. Unabhängig davon, ob beide Anzeigeeinrichtungen als Farbdisplay ausgeführt sind, oder ob nur die erste Anzeigeeinrichtung ein Farbdisplay ist, kann die Information durch komplette Einfärbung des Displayhintergrunds, also der Grundfarbe, visualisiert werden. Auf dieser farbigen Hintergrundfläche können selbstverständlich auch weitere Textinformationen, Symbole und Ähnliches angezeigt werden, insbesondere an der ersten Anzeigeeinrichtung, wenn noch zusätzliche Informationen wie Trittfrequenz etc. dargestellt werden sollen.

Eine gleiche Information kann an beiden Displays, unabhängig davon, ob es nun normale Schwarz-Weiß-Displays oder Farbdisplays sind, auch durch Buchstaben, Texte oder Symbole dargestellt werden. Beispielsweise kann eine bestimmte Trainingszone auch durch einen entsprechenden Buchstaben visualisiert werden, beispielsweise durch die Buchstaben A - E bei fünf Trainingszonen, oder etwaige andere Symbole. Natürlich ist auch eine kumulative Darstellung gleicher Informationen möglich, wobei eine erste gleiche Information durch eine gleiche Farbkodierung und eine zweite Information durch gleiche Symbole, Buchstaben oder Zahlen dargestellt wird.

Darüber hinaus kann eine Information auch beispielsweise durch eine Blinkanzeige dargestellt werden, insbesondere wenn die erste Anzeigeeinrichtung ein Farbdisplay ist und die zweite Anzeigeeinrichtung mehrere Lichtquellen, insbesondere LEDs umfasst. In diesem Fall kann eine Information durch eine blinkende Darstellung am Farbdisplay (hierbei kann der gesamte Hintergrund blinken, oder nur ein bestimmter Darstellungsabschnitt am Display), und ein Blinken der Lichtquelle (ein-aus) dargestellt werden. Hinter dem Blinken kann letztlich eine weitere Information stehen, beispielsweise eine Warninformation, die dem Trainierenden und dem Trainer gegeben wird, beispielsweise wenn sich eine Trainingszone verändert, um die beiden angesprochenen Personen sofort auf die Veränderung aufmerksam zu machen. Das heißt, dass zum einen die Farbe die entsprechende Trainingszone markiert, wie der Blinkbetrieb die Zonenveränderung visualisiert.

Die erste Anzeigeeinrichtung ist am Übungsgerät in an sich bekannter Weise zu befestigen, sie findet sich beispielsweise bei einem Trainingsrad im Bereich des Lenkers. Die zweite Anzeigeeinrichtung ist an einer geeigneten Vertikal- oder Horizontalstrebe, die das Gestell aufweist, angeordnet, beispielsweise mittels eines geeigneten Halters, oder durch Integration, also festes Einsetzen in diese Strebe. Ist beispielsweise ein Display vorgesehen, so wird dieses zum Beispiel in eine entsprechende Aussparung an der Strebe fest eingebaut, Gleiches gilt für etwaige LEDs oder dergleichen, sodass es möglich ist, die entsprechenden Zuleitungen im Inneren der hohlen Strebe zur geräteseitigen, zentralen Recheneinrichtung, an der sämtliche von den beliebigen Sensoren erfassten Messwerte zusammenlaufen, zu führen.

Alternativ dazu ist es möglich, die erste Anzeigeeinrichtung, die zweite Anzeigeeinrichtung und die Recheneinrichtung an bzw. in einem gemeinsamen Gehäuse anzuordnen, das vorzugsweise am Lenker befestigt ist. Alle relevanten Baugruppen sind folglich zu einer kompakten und einfach handzuhabenden Einheit infolge der Integration an bzw. in einem gemeinsamen Gehäuse verbunden.

Weitere Vorteile, Merkmale und Einzelheiten der Erfindung ergeben sich aus den im Folgenden beschriebenen Ausführungsbeispielen sowie anhand der Zeichnung. Dabei zeigen:
- Fig. 1: eine Perspektivansicht eines erfindungsgemäßen Übungsgeräts in Form eines Trainingsrads mit Blick auf die erste Anzeigeeinrichtung,
- Fig. 2: eine zweite Perspektivansicht des Übungsgeräts aus Fig. 1 mit Blick auf die Vorderseite und die dortige zweite Anzeigeeinrichtung,
- Fig. 3: eine Teilansicht eines Übungsgeräts zur Darstellung einer weiteren Ausführungsform der zweiten Anzeigeeinrichtung in Form einzelner LEDs, und
- Fig. 4: eine Perspektivansicht entsprechend Fig. 2, wobei die erste Anzeigeeinrichtung, die zweite Anzeigeeinrichtung und die Recheneinrichtung an bzw. in einem gemeinsamen Gehäuse vorgesehen sind.

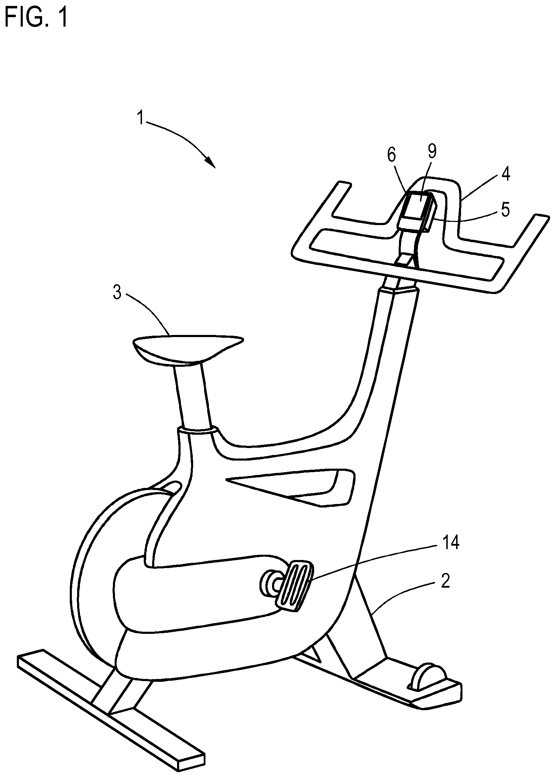

Fig. 1 zeigt ein erfindungsgemäßes stationäres Übungsgerät 1 hier in Form eines Trainingsrades, umfassend ein Gestell 2 mit einem Sattel 3, auf dem der Übende Platz nimmt, sowie einem Lenker 4 und einer Pedalerie 14, über die in an sich bekannter Weise der Übende eine Bewegungseinheit antreibt, die einen Widerstand entgegensetzt, den er zu Trainingszwecken zu überwinden hat.

Vorgesehen ist ferner eine Recheneinrichtung 5 mit zugeordneter Anzeigeeinrichtung 6. Die Recheneinrichtung 5 kommuniziert mit einem oder mehreren Sensoren, die der Erfassung unterschiedlicher Messwerte dienen, gestützt auf welche sodann auszugebende Informationen ermittelt werden. Üblicherweise befindet sich ein oder befinden sich mehrere Sensoren im Bereich des Tretlagers der Pedalerie 14, also letztlich im Bereich der Bewegungseinheit. Über diese dortigen Sensoren werden gerätebezogene Messwerte aufgenommen, wie insbesondere die Trittfrequenz respektive Drehzahl, die beim Treten erzeugte Leistung, sowie der Widerstandslevel, der dem Treten seitens der Bewegungseinheit entgegengesetzt wird, beispielsweise durch Einstellung einer bestimmten Übersetzung oder eines Ganges oder einer Steigung etc. All diese, die über geeignete Sensoren wie Drehzahlmesser, Dehnungsmessstreifen, etc. ermittelt werden, werden z. B. über Kabel an die Recheneinrichtung 5 gegeben.

Vorgesehen ist des Weiteren ein Brustgurt 7, den der Übende trägt. Auch an diesem befindet sich ein hier nur exemplarisch dargestellter Sensor 8, der als personenbezogener Messwert kontinuierlich die Ist-Herzfrequenz des Übenden misst. Dieser Sensor 8 kommuniziert mit der Recheneinrichtung, sei es über ein Kabel, sei es kabellos, also per Funk. Das heißt, dass folglich recheneinrichtungsseitig sowohl gerätebezogene Messwerte als auch personenbezogene Messwerte vorliegen.

Um eine möglichst optimale Berechnung der anzuzeigenden Trainingsdaten zu erzielen, benötigt die Recheneinrichtung ferner die Eingabe von Benutzerdaten durch den Übenden selbst. Dies geschieht bevorzugt unmittelbar über die Anzeigeeinrichtung 6, die hierzu als Touchscreen ausgebildet ist und entsprechend mit der Recheneinrichtung 5 kommuniziert. Die einzugebenden Benutzerdaten dienen der Individualisierung der Berechnungsgrundlage für die auszugebenden Informationen, um ein effizientes und personengerechtes Training zu ermöglichen. Beispielsweise werden folgende Benutzerdaten manuell eingegeben (sofern bekannt, nicht abschließend): maximale Herzfrequenz, Geschlecht, Alter, Gewicht, Trainingsaktivität, aerobe/anaerobe Schwelle, FTP-Wert (die maximale Leistung, die ein Fahrer im anaeroben/aeroben Stoffwechsel eine Stunde lang leisten kann), Watt/kg Körpergewicht an der anaeroben Schwelle, Trainingszonen (für Herzfrequenz oder Watt-basiertes Training).

Je mehr Benutzerdaten und Messwerte recheneinrichtungsseitig vorliegen, desto mehr respektive genauer kann die Berechnung der auszugebenden Informationen und damit die Trainingssteuerung optimiert werden. Anhand der Benutzerdaten und Messwerte können beispielsweise folgende Parameter dargestellt respektive ermittelt werden:
- Herzfrequenz aktuell
- Herzfrequenz maximal
- Trittfrequenz (Drehzahl) aktuell
- Trittfrequenz (Drehzahl) maximal
- Leistung (Watt) aktuell
- Leistung (Watt) maximal
- Trainingsdauer
- Widerstandslevel aktuell
- Widerstandslevel maximal
- Herzfrequenzdurchschnitt
- Trittfrequenz- (Drehzahl)-Durchschnitt
- Leistungsdurchschnitt (Watt)
- Widerstandslevel-Durchschnitt
- Entfernung
- Geschwindigkeit
- Prozentwert der Herzfrequenz in Relation zur maximalen Herzfrequenz
- Trainingszonen für Herzfrequenz in Relation zur maximalen Herzfrequenz
- Prozentwert Leistung (Watt) in Relation zu einem Schwellwert (Indexwert)
- Trainingszonen für Leistung (Watt) in Relation zu einem Indexwert

Die genannten Parameter sind nicht abschließend.

Unter diesen Parametern ist insbesondere der Prozentwert der Herzfrequenz und daraus resultierend die Trainingszone bezogen auf die Herzfrequenz ein sehr wichtiger Parameter. Denn die gegebene Herzfrequenz stellt ein unmittelbares Maß für die Belastung des Übenden dar.

Zum einen wird die Belastung in fünf Trainingszonen unterteilt. Diese Trainingszonen ergeben sich als prozentuale Intervalle der maximalen Herzfrequenz, die sich wiederum aus den benutzerspezifischen Parametern (Geschlecht, Alter, Gewicht, Trainingszustand etc.) ergibt. Die einzelnen Trainingszonen sind genähert:
50 - 60 % MHF: Gesundheitszone
60 - 70 % MHF: Verbrennungszone
70 - 80 % MHF: Aerobe Zone
80 - 90 % MHF: Anaerobe Zone
90 - 100 % MHF: Rote Zone

### MHF = maximale Herzfrequenz

Jeder Zone ist ein zonenspezifischer Trainingsinhalt zugeordnet. So ist die Gesundheitszone eine Einstiegs-/Reha-Belastungszone, die der Stabilisierung des Herz-Kreislauf-Systems dient, wie auch der Regeneration, die subjektive Belastung ist sehr leicht bis leicht. In der Fettverbrennungszone findet eine optimale Fettverbrennung für geübtere Sportler statt, sie dient der weiteren Kräftigung des Herz-Kreislauf-Systems. Die subjektive Belastung ist leicht bis mittel. In der aeroben Zone erfolgt eine Steigerung der Ausdauer und Kräftigung des Herzens und Ökonomisierung des Herz-Kreislauf- und Atmungssystems wie auch eine Verbesserung der aeroben Kapazität. Auch findet eine Erhöhung der Fettverbrennungsrate statt. Die subjektive Belastung ist mittel bis anstrengend. In der anaeroben Zone findet eine Verschiebung der anaeroben Schwelle nach oben statt, sie dient der Verbesserung des Milchsäureabbaus, die subjektive Belastung ist anstrengend bis sehr anstrengend. Die "Rote Zone" hingegen ist nur für professionelle Hochleistungssportler geeignet, hier findet eine maximale Belastung am absoluten Leistungslimit statt. Die subjektive Belastung ist extrem anstrengend.

Diese Trainingszonen werden nun, gestützt auf die subjektiven, personenbezogenen Daten, personenindividuell ermittelt. Nachdem wie beschrieben über den Sensor 8 während des Trainings auch kontinuierlich die Ist-Herzfrequenz ermittelt wird, wird folglich kontinuierlich überprüft, in welcher Trainingszone der Übende gerade trainiert. Die gerade "bearbeitete" Trainingszone wird nun an der ersten Anzeigeeinrichtung 6 visualisiert. Diese Anzeigeeinrichtung 6 ist hierzu bevorzugt als Farbdisplay 9 ausgeführt, an dem beliebige Farben dargestellt werden können.

Bevorzugt ist nun jeder einzelnen der fünf Zonen eine bestimmte Farbe zugeordnet. Die Gesundheitszone wird beispielsweise mit der Farbe Weiß, die Fettverbrennungszone mit Blau, die aerobe Zone mit Grün, die anaerobe Zone mit Gelb und die "Rote Zone" mit Rot farbkodiert dargestellt. Diese Farbdarstellung erfolgt erfindungsgemäß bevorzugt derart, dass in der entsprechenden, zonenbezogenen Farbe die gesamte Displayfläche ausgelegt wird, das heißt, dass die Hintergrundfarbe des Displays der entsprechenden Zonenfarbe entspricht. Das heißt, dass der Übende sofort anhand der Displayhintergrundfarbe erkennt, in welcher Zone er gerade trainiert, sodass er sich selbst kontrollieren kann.

Zeitgleich werden natürlich auch entsprechende weitere Informationen, die entweder unmittelbar gemessen werden (z. B. aktuelle Trittfrequenz, Leistung, Widerstand etc.) oder errechnet werden (z. B. durchschnittliche Trittfrequenz, Durchschnittsleistungen etc.) auf dem großflächigen Display visualisiert.

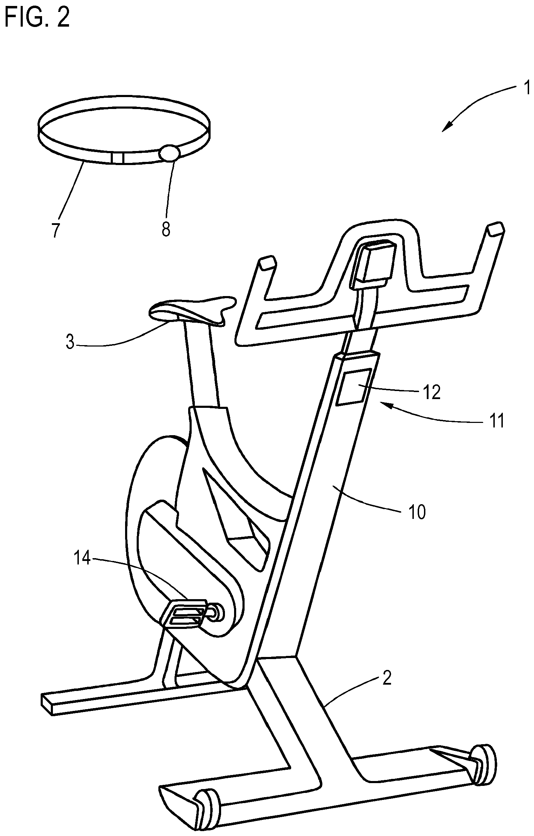

Wie Fig. 2 zeigt, ist an einer Vertikalstrebe 10 des Gestells 2 eine gegebenenfalls separat ein- und ausschaltbare zweite Anzeigeeinrichtung 11, auch hier in Form eines Farbdisplays 12, angeordnet, vorzugsweise integriert. Während die erste Anzeigeeinrichtung 6, also das Farbdisplay 9, zum Übenden hin gerichtet ist, ist die zweite Anzeigeeinrichtung 11 respektive das Farbdisplay 12 in die entgegengesetzte Richtung gerichtet, mithin also zu einem Trainer, der vor dem Übungsgerät steht respektive sich davor bewegt, wenn im Rahmen eines Gruppenkurses mehrere Teilnehmer gleichzeitig trainieren. Dieses Farbdisplay 12 kommuniziert ebenfalls mit der Recheneinrichtung 5, das heißt, dass grundsätzlich auch an ihm beliebige Informationen, wie sie auch am ersten Farbdisplay 9 dargestellt werden, dargestellt werden können.

Hierüber erfolgt also eine Informationsgabe direkt an den Trainer selbst, wobei am Farbdisplay 12 jedoch nicht alle am Farbdisplay 9 angezeigten Informationen darzustellen sind. Vielmehr ist es dort lediglich erforderlich, die wesentlichen Informationen zu visualisieren, sodass der Trainer sehr rasch eine Informationserfassung vornehmen und etwaige Trainingskommandos geben kann.

Wie ausgeführt ist eine der wesentlichen Informationen die gerade "bearbeitete" Trainingszone, die letztlich den individuellen Leistungs- respektive Belastungszustand des Trainierenden darstellt. Diese farbkodierte Trainingszone wird nun großflächig an dem Farbdisplay 12 angezeigt. Das Farbdisplay 12 leuchtet in derselben Farbe, wie auch das Farbdisplay 9 mit seiner Hintergrundfarbe ausgeleuchtet ist. Das heißt, dass simultan beide Farbdisplays 9 und 12 in der gleichen Farbe leuchten, sodass sowohl der Trainierende als auch der auf der anderen Seite stehende Trainer durch einen einfachen Blick auf das jeweilige Display die jeweilige farbkodierte Trainingszone erkennen kann. Da das Farbdisplay 12 relativ großflächig ist, ist es ohne Weiteres möglich, dass der Trainer die jeweilige farbkodierte Trainingszone auch aus einer beachtlichen Entfernung erkennen kann.

Dies ermöglicht es dem Trainer, auch wenn er abseits steht, beispielsweise einen Wechsel von einer aeroben Zone, in der der Übende eigentlich "arbeiten" sollte, zu einer anaeroben Zone, die aus gesundheitlichen Gründen für ihn nachteilig ist, zu erkennen. Er kann daraufhin sofort ein entsprechendes Kommando geben (z. B. die Trittfrequenz zu verringern oder den Widerstand zu reduzieren), um so das Training gesundheitsoptimiert zu steuern.

Sowohl das erste als auch insbesondere das zweite Farbdisplay 12 kann darüber hinaus beispielsweise auch in einem Blinkbetrieb arbeiten, beispielsweise dann, wenn ein eben geschilderter Zonenwechsel gegeben ist. Ein solches Blinken ist noch auffälliger, wird also vom Trainer (und, wenn dies auch an dem ersten Farbdisplay 9 der Fall ist, vom Trainierenden) sofort erkannt, sodass Gegenmaßnahmen ergriffen werden können.

An dieser Stelle ist darauf hinzuweisen, dass anstelle einer Farbkodierung natürlich auch die Zonen in entsprechenden anderen Symbolen individualisiert werden können. So können die einzelnen Zonen beispielsweise mit Buchstaben A - E (A = Gesundheitszone, E = Rote Zone) visualisiert werden. Diese Buchstaben lassen sich auch insbesondere an dem zweiten Farbdisplay 12 großflächig darstellen, werden also sofort erkannt. Handelt es sich um ein Farbdisplay, kann darüber hinaus sogar eine Farbdarstellung der einzelnen Buchstaben erfolgen, das heißt, dass dann eine Doppelkodierung gegeben wäre.

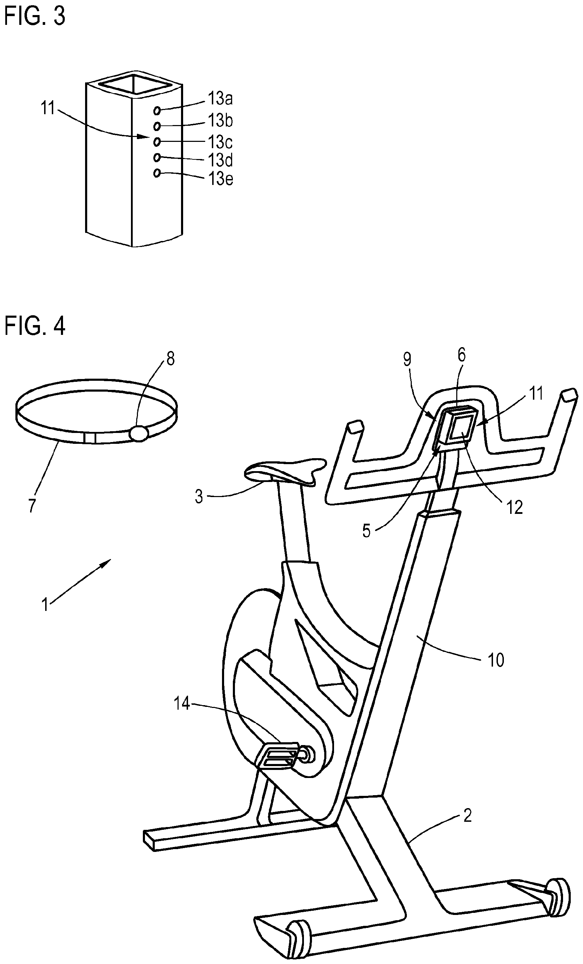

Fig. 3 zeigt schließlich eine weitere Ausführungsform einer zweiten Anzeigeeinrichtung 11. Diese ist hier in Form mehrerer separater und einzeln ansteuerbarer Lichtquellen 13a - 13e ausgeführt. Bei den Lichtquellen handelt es sich beispielsweise um LEDs. Wichtig ist, dass die einzelnen Lichtquellen unterschiedlich farbig sind, sodass wiederum über jeweils eine Lichtquelle respektive LED eine bestimmte, farbkodierte zugeordnete Trainingszone visualisiert werden kann. Beispielsweise ist die Lichtquelle 13a als Weißlichtquelle zur Visualisierung der Gesundheitszone vorgesehen, die Lichtquelle 13b emittiert blaues Licht und ist der Fettverbrennungszone zugeordnet, die Lichtquelle 13c emittiert grünes Licht und ist der aeroben Zone zugeordnet, die Lichtquelle 13d emittiert gelbes Licht und ist der anaeroben Zone zugeordnet, während die Lichtquelle 13e rotes Licht emittiert und der "Roten Zone" zugeordnet ist. Je nachdem welche Zone also gerade "bearbeitet" wird, leuchtet folglich sowohl einerseits die Hintergrundfarbe an dem ersten Farbdisplay 9 auf, zeitgleich aber auch die entsprechende, farblich zugeordnete Lichtquelle 13a - 13e.

Grundsätzlich ist es selbstverständlich auch denkbar, die einzelnen Lichtquellen 13a - 13e so auszugestalten, dass jede Lichtquelle eine beliebige Farbe emittieren kann, beispielsweise durch Verbau von entsprechenden LED-Gruppen (RGB-Gruppen), das heißt, dass beliebige Farben durch Lichtmischen an der jeweiligen Lichtquelle erzeugt werden können. Dies ermöglicht es wiederum, dass alle Lichtquellen in der gleichen Farbe emittieren, wobei die jeweilige Farbe jedoch einstellbar ist. Es ergibt sich also ein Lichtband, über das die jeweilige Trainingszone charakterisiert wird.

Wiederum ist auch bei dieser Ausgestaltung die Möglichkeit einer Blinkanzeige gegeben, beispielsweise bei einem Wechsel von einer Zone zu einer anderen, also bei einem Wechsel von einer Farbe zu einer anderen.

Fig. 4 zeigt schließlich ein Übungsgerät 1 in Form eine Trainingsrads, wie es aus Fig. 1 und 2 bereits bekannt ist. Auf die diesbezüglichen Ausführungen wird verwiesen. Anders als bei der dortigen Ausführungsform sind bei dieser Ausgestaltung die erste Anzeigeeinrichtung 6, vorzugsweise wieder in Form eines Farbdisplays 9, gegebenenfalls in Touchscreen-Ausführung, die zweite Anzeigeeinrichtung 11, vorzugsweise ebenfalls in Form eines Farbdisplays, und die Recheneinrichtung 5 an bzw. in einem gemeinsamen Gehäuse angeordnet, das lenkerseitig verbaut ist. Da üblicherweise eine Recheneinrichtung ein entsprechendes Gehäuse aufweist, in dem die erforderlichen Komponenten untergebracht sind, bietet es sich an, an diesem die beiden Anzeigeeinrichtungen 6 und 11 anzubringen, wobei die eine wiederum zum Trainierenden gerichtet ist, während die andere zur entgegengesetzten Seite gerichtet ist. Statt eines Farbdisplays können natürlich auch hier, insbesondere als zweite Anzeigeeinrichtung, auch einzelne unterschiedliche Farb-LEDs vorgesehen sein. Diese können auch im Gehäuse integriert sein und eine transparente Abdeckung des Gehäuses hinterleuchten, so dass betrachterseitig eine entsprechend größerflächige Farbdarstellung sichtbar ist.

## Patentansprüche

1. Stationäres Übungsgerät für einen einzelnen Übenden zur körperlichen Ertüchtigung, insbesondere Trainingsrad, umfassend ein Gestell mit einer vom Übenden zu bewegenden oder selbst angetriebenen und mit dem Übenden zusammenwirkenden Bewegungseinheit, einen oder mehrere der Bewegungseinheit und/oder dem Übenden zugeordnete Sensoren zur Erfassung von Messwerten, sowie eine Recheneinrichtung zur Ermittlung einer oder mehrerer messwertbezogener Informationen, die an einer gestellseitigen Anzeigeeinrichtung ausgegeben werden, **dadurch gekennzeichnet, dass** eine erste Anzeigeeinrichtung (6) vorgesehen ist, die zur Anzeige einer oder mehrerer messwertbezogener Informationen zum Übenden gerichtet ist, und dass eine zweite Anzeigeeinrichtung (11) vorgesehen ist, die zur Ausgabe wenigstens einer messwertbezogenen Information zur gegenüberliegenden Seite gerichtet ist, wobei eine gleiche messwertbezogene Information an der ersten und der zweiten Anzeigeeinrichtung (6, 11) simultan ausgebbar ist, wobei an der ersten und der zweiten Anzeigeeinrichtung (6, 11) eine Herzfrequenz und/oder eine Trittfrequenz und/oder eine Leistung und/oder ein Prozentwert in Relation zur maximalen Herzfrequenz und/oder eine Trainingszone bezogen auf die Herzfrequenz darstellbar sind.

2. Übungsgerät nach Anspruch 1, **dadurch gekennzeichnet, dass** über die erste und die zweite Anzeigeeinrichtung (6, 11) simultan mehrere gleiche Informationen darstellbar sind.

3. Übungsgerät nach einem der vorangehenden Ansprüche, **dadurch gekennzeichnet, dass** die erste Anzeigeeinrichtung (6) ein Display (9), insbesondere ein Farbdisplay, oder mehrere in unterschiedlichen Farben leuchtende Lichtquellen, insbesondere LEDs, aufweist, ist.

4. Übungsgerät nach einem der vorangehenden Ansprüche, **dadurch gekennzeichnet, dass** die zweite Anzeigeeinrichtung (11) ein Display (12), insbesondere ein Farbdisplay ist, oder dass die zweite Anzeigeeinrichtung (11) mehrere in unterschiedlichen Farben leuchtende Lichtquellen (13a - 13e), insbesondere LEDs aufweist.

5. Übungsgerät nach Anspruch 3 oder 4, **dadurch gekennzeichnet, dass** eine gleiche Information, die an der ersten und der zweiten Anzeigeeinrichtung (6, 11) simultan anzeigbar ist, in Form einer gleichen Farbdarstellung an beiden Farbdisplays (9, 12) oder einer gleichen Farbdarstellung am Farbdisplay (9) und der entsprechenden Lichtquelle (13a - 13e) ausgebbar ist.

6. Übungsgerät nach Anspruch 5, **dadurch gekennzeichnet, dass** als Farbdarstellung an einem Farbdisplay (9, 12) der Displayhintergrund farbig dargestellt wird.

7. Übungsgerät nach einem der vorangehenden Ansprüche, **dadurch gekennzeichnet, dass** eine gleiche Information an beiden Anzeigeeinrichtungen (6, 11) durch Buchstaben, Text oder Symbole darstellbar ist.

8. Übungsgerät nach einem der Ansprüche 3 bis 6, **dadurch gekennzeichnet, dass** die erste Anzeigeeinrichtung (6) ein Farbdisplay (9) ist und die zweite Anzeigeeinrichtung (11) mehrere Lichtquellen (13a - 13e), insbesondere LEDs umfasst, oder dass beide Anzeigeeinrichtungen (6, 11) Farbdisplays (9, 12) sind, wobei eine gleiche Information durch eine blinkende Darstellung am Farbdisplay und ein Blinken der Lichtquelle oder eine blinkende Darstellung an beiden Farbdisplays (9, 12) ausgebbar ist.

9. Übungsgerät nach einem der vorangehenden Ansprüche, **dadurch gekennzeichnet, dass** das Gestell (2) wenigstens eine Vertikal- oder wenigstens eine Horizontalstrebe (10) aufweist, an der die zweite Anzeigeeinrichtung (11) angeordnet ist.

10. Übungsgerät nach Anspruch 9, **dadurch gekennzeichnet, dass** die zweite Anzeigeeinrichtung (11) in die Vertikal- oder Horizontalstrebe (10) integriert ist.

11. Übungsgerät nach einem der Ansprüche 1 bis 8, **dadurch gekennzeichnet, dass** die erste Anzeigeeinrichtung (6), die zweite Anzeigeeinrichtung (11) und die Recheneinrichtung (5) an bzw. in einem gemeinsamen Gehäuse angeordnet sind.

## Claims

1. Stationary training device for a single exerciser for physical training, in particular an exercise bicycle, comprising a frame with a movement unit that should be moved by the exerciser or that is self-driven and interacts with the exerciser, one or more sensors that are assigned to the movement unit and/or the exerciser for capturing measurement values, and a computer apparatus for ascertaining one or more measurement-value-related information items which are output on a frame-side indicating apparatus, **characterized in that** provision is made of a first indicating apparatus (6) that is directed to display one or more measurement-value-related information items to the exerciser and **in that** provision is made of a second indicating apparatus (11) that is directed to output at least a measurement-value-related information item to the opposite side, wherein the same measurement-value-related information item can be output simultaneously at the first indicating apparatus (6) and the second indicating apparatus (11), wherein a heart rate and/or a cadence and/or power and/or a percentage value in relation to the maximum heart rate and/or a training zone in relation to the heart rate are displayable at the first indicating apparatus (6) and the second indicating apparatus (11).

2. Training device according to Claim 1, **characterized in that** a plurality of the same information items are displayable simultaneously by way of the first indicating apparatus (6) and the second indicating apparatus (11).

3. Training device according to either of the preceding claims, **characterized in that** the first indicating apparatus (6) is a display (9), in particular a colour display, or has a plurality of light sources, in particular LEDs, that shine in different colours.

4. Training device according to any one of the preceding claims, **characterized in that** the second indicating apparatus (11) is a display (12), in particular a colour display, or **in that** the second indicating apparatus (11) has a plurality of light sources (13a-13e), in particular LEDs, that shine in different colours.

5. Training device according to Claim 3 or 4, **characterized in that** the same information item that is displayable simultaneously at the first indicating apparatus (6) and the second indicating apparatus (11) can be output in the form of the same colour representation on both colour displays (9, 12) or the same colour representation on the colour display (9) and the corresponding light source (13a-13e).

6. Training device according to Claim 5, **characterized in that**, as a colour representation on a colour display (9, 12), the display background is displayed in colour.

7. Training device according to any one of the preceding claims, **characterized in that** the same information item is displayable on both indicating apparatuses (6, 11) by letters, text or symbols.

8. Training device according to any one of Claims 3 to 6, **characterized in that** the first indicating apparatus (6) is a colour display (9) and the second indicating apparatus (11) comprises a plurality of light sources (13a-13e), in particular LEDs, or **in that** both indicating apparatuses (6, 11) are colour displays (9, 12), wherein the same information item can be output by a blinking representation on the colour display and a blinking of the light source or a blinking representation on both colour displays (9, 12).

9. Training device according to any one of the preceding claims, **characterized in that** the frame (2) has at least a vertical strut or at least a horizontal strut (10), on which the second indicating apparatus (11) is arranged.

10. Training device according to Claim 9, **characterized in that** the second indicating apparatus (11) is integrated into the vertical or horizontal strut (10).

11. Training device according to any one of Claims 1 to 8, **characterized in that** the first indicating apparatus (6), the second indicating apparatus (11) and the computer apparatus (5) are arranged at or in a common casing.

## Revendications

1. Appareil d'exercice fixe pour un pratiquant individuel servant à l'entraînement physique, notamment vélo d'appartement, comprenant un bâti muni d'une unité à mouvement à mettre en mouvement par le pratiquant ou auto-entraînée et coopérant avec le pratiquant, un ou plusieurs capteurs associés à l'unité à mouvement et/ou au pratiquant destinés à acquérir des valeurs mesurées, ainsi qu'un dispositif de calcul destiné à déterminer une ou plusieurs informations en rapport avec les valeurs mesurées, lesquelles sont délivrées sur un dispositif d'affichage du côté du bâti, **caractérisé en ce qu'**il existe un premier dispositif d'affichage (6) qui est orienté vers le pratiquant en vue d'afficher une ou plusieurs informations en rapport avec les valeurs mesurées, et **en ce qu'**il existe un deuxième dispositif d'affichage (11) qui est orienté vers le côté opposé en vue de délivrer au moins une information en rapport avec les valeurs mesurées, une information en rapport avec les valeurs mesurées identique pouvant être délivrée simultanément au niveau du premier et du deuxième dispositif d'affichage (6, 11), une fréquence cardiaque et/ou une fréquence de pas et/ou une puissance et/ou un pourcentage en relation avec la fréquence cardiaque maximale et/ou une zone d'exercice en rapport avec la fréquence cardiaque pouvant être représentées au niveau du premier et du deuxième dispositif d'affichage (6, 11).

2. Appareil d'exercice selon la revendication 1, **caractérisé en ce que** plusieurs mêmes informations peuvent être représentées simultanément par le biais du premier et du deuxième dispositif d'affichage (6, 11).

3. Appareil d'exercice selon l'une des revendications précédentes, **caractérisé en ce que** le premier dispositif d'affichage (6) est un afficheur (9), notamment un afficheur en couleurs, ou plusieurs sources de lumière éclairant dans des couleurs différentes, notamment des LED.

4. Appareil d'exercice selon l'une des revendications précédentes, **caractérisé en ce que** le deuxième dispositif d'affichage (11) est un afficheur (12), notamment un afficheur en couleurs, ou **en ce que** le deuxième dispositif d'affichage (11) possède plusieurs sources de lumière (13a - 13e) éclairant dans des couleurs différentes, notamment des LED.

5. Appareil d'exercice selon la revendication 3 ou 4, **caractérisé en ce qu'**une même information peut être affichée simultanément sur le premier et le deuxième dispositif d'affichage (6, 11), sous la forme d'une même représentation en couleur sur les deux afficheurs en couleurs (9, 12) ou une même représentation en couleur pouvant être délivrée sur l'afficheur en couleurs (9) et la source de lumière (13a - 13e) correspondante.

6. Appareil d'exercice selon la revendication 5, **caractérisé en ce que** la représentation en couleur représentée sur un afficheur en couleurs (9, 12) est la représentation en couleur de l'arrière-plan de l'afficheur.

7. Appareil d'exercice selon l'une des revendications précédentes, **caractérisé en ce que** la même information peut être représentée sur les deux dispositifs d'affichage (6, 11) par des lettres, du texte ou des symboles.

8. Appareil d'exercice selon l'une des revendications 3 à 6, **caractérisé en ce que** le premier dispositif d'affichage (6) est un afficheur en couleurs (9) et le deuxième dispositif d'affichage (11) comprend plusieurs sources de lumière (13a - 13e), notamment des LED, ou **en ce que** les deux dispositifs d'affichage (6, 11) sont des afficheurs en couleurs (9, 12), une même information pouvant être délivrée par une représentation clignotante sur l'afficheur en couleurs et un clignotement de la source de lumière ou une représentation clignotante sur les deux afficheurs en couleurs (9, 12).

9. Appareil d'exercice selon l'une des revendications précédentes, **caractérisé en ce que** le bâti (2) possède au moins une barre de renfort verticale ou au moins une barre de renfort horizontale (10) sur laquelle est disposé le deuxième dispositif d'affichage (11).

10. Appareil d'exercice selon la revendication 9, **caractérisé en ce que** le deuxième dispositif d'affichage (11) est intégré dans la barre de renfort verticale ou horizontale (10).

11. Appareil d'exercice selon l'une des revendications 1 à 8, **caractérisé en ce que** le premier dispositif d'affichage (6), le deuxième dispositif d'affichage (11) et le dispositif de calcul (5) sont disposés sur ou dans un boîtier commun.
